# EUROPEAN PATENT APPLICATION

(11) **EP 1 000 613 A2**
(43) Date of publication of application: **17.05.2000**
(21) Application number: 99402170.7
(22) Date of filing: 01.09.1999
(51) Int. Cl.: A61K 7/48

(54) **Composition against the ageing and its use**

(30) Priority: 04.09.1998 FR 9811080
(71) Applicant: ROC, 92130 Issy les Moulineaux (FR)
(72) Inventor: Castelli, Dominique, 75017 Paris (FR); Ries, Gerd, 47259 Duisburg-Huckingen (DE)
(74) Representative: Martin, Jean-Jacques

(57) **Abstract**

The present invention relates to a composition intended to combat the effects of ageing of the skin and/or the exoskeleton, to the use of such a composition and to a method for the cosmetic treatment of changes associated with ageing of the skin and/or the exoskeleton.

In particular, the present invention relates to a cosmetic composition, characterized in that it contains a combination of at least one retinoid chosen from retinol, esters thereof and retinaldehydes, with at least one plant hormone chosen from the group consisting of phyto-sterols and ethoxylated derivatives thereof, pentacyclic triterpenes, isoflavones, isoflavone glucosides, coumestanes and saponosides, and at least one cosmetically acceptable excipient.

This composition is particularly intended to improve the elasticity of the skin and/or is intended for the prevention or treatment of changes in the skin and the exoskeleton associated with ageing.

## Description

The present invention relates to a composition intended to combat the effects of ageing of the skin and/or the exoskeleton, to the use of such a composition and to a method for the cosmetic treatment of changes associated with ageing of the skin and/or the exoskeleton.

The skin comprises three compartments, the epidermis, the outer layer of which is the stratum corneum, the dermis and, deeper down, the hypodermis. Exchanges exist between these various dermal and epidermal compartments, which are intended to ensure cell renewal and the cohesion and moisturization of the outer layers.

The dermis results from the biosynthetic activity of fibroblasts, which develop the constituents of the extracellular matrix. This matrix is formed of four major families of macromolecules: collagens, elastin, structural glycoproteins and proteoglycans.

It is known that ageing is a physiological phenomenon which follows a period of growth. It is reflected in particular by thinning of the skin and a loss of elasticity, leading to the appearance of more or less deep wrinkles; there is also drying-out of the surface and anarchic pigmentation can also be observed.

More particularly, it is known that the ageing process involves an increase in the content of a specific class of enzymes, namely matrix metalloproteinases, in particular the MMP-1 collagenases and the MMP-3 stromelysines, as well as a decrease in the content of tissue compounds which inhibit the activity of metalloproteinases, referred to hereinbelow as "TIMP inhibitors" (corresponding to "tissue inhibitor of metalloproteinase"). These phenomena associated with ageing are reflected by a gradual destruction of the extracellular matrix and thus in particular by the appearance of the visible signs of ageing of the skin.

The article by C. Mauch et al., "Role of the Extracellular Matrix in the Degradation of Connective Tissue", Arch. Dermatol. Res. (1994), 287: 107-114, may be read in this respect, in particular as regards TIMP inhibitors.

Many active agents have been proposed for preventing or delaying the effects of ageing. It has been shown in particular that retinoids have a favourable effect on improving the appearance and condition of the skin and combating the signs of ageing.

It has now been found, entirely surprisingly and unexpectedly, that the use of a combination of certain retinoids with a specific class of plant hormones makes it possible to potentiate the activity of the retinoid used, or even to provide a synergy effect, in order visibly to counter the effects associated with ageing of the skin and/or the exoskeleton.

More particularly, the use of such a combination makes it possible to potentiate the activity of the retinoid, or even to provide a synergy effect, in order to increase the content of TIMP-1 inhibitors and thus to counter the destruction of the extracellular matrix associated with ageing as explained above.

This result is all the more surprising since the use of such a combination does not entail a significant effect of increasing the content of metalloproteinases, which a person skilled in the art might however have expected, in the context of a phenomenon of feedback regulation in response to the increase in the content of TIMP inhibitors.

This result is moreover surprising since the use of such a combination does not entail a significant decrease in cell growth (or proliferation). Indeed, a person skilled in the art might also have expected that a decrease in the activity of metalloproteinases, induced by increasing the content of TIMP inhibitors, would entail a decrease in the movement of cells, thus resulting in contact inhibition of cellular division and consequently an undesirable decrease in cell renewal.

In other words, the retinoid-plant hormone combination according to the invention makes it advantageously possible to counter the ageing process associated with the activity of metalloproteinases without, however, undesirably favouring any other ageing process associated with a decrease in cell renewal.

The present invention thus relates to a cosmetic composition, characterized in that it contains a combination of at least one retinoid chosen from retinol, esters thereof and retinaldehydes, with at least one plant hormone chosen from the group consisting of phytosterols and ethoxylated derivatives thereof, pentacyclic triterpenes, isoflavones, isoflavone glucosides, coumestanes and saponosides, and at least one cosmetically acceptable excipient.

As regards the retinoid which can be used in the composition according to the invention, retinol and its esters are more particularly preferred.

The term "retinol" should be understood as including the hydrogenated and non-hydrogenated isomers, such as 9-cis-retinol and didehydroretinol.

The retinol esters which can be used as retinoids in the composition according to the invention are, in particular, the esters obtained with acetic acid, propionic acid, palmitic acid or stearic acid.

Moreover, the expression "retinaldehydes" should be understood as including the four stereoisomeric forms trans, 13-cis, 11-cis and 9-cis.

As regards the plant hormone which can be used in the composition according to the invention, phytosterols and the ethoxylated derivatives thereof are more particularly preferred.

Among these, mention may be made in particular of β-sitosterol, α-sitosterol, γ-sitosterol, β-sitostanol, stigmasterol, stigmastanol, campesterol or campestanol. For example, β-sitosterol can be used in the form of the product referred to as "Ultra" (mainly comprising β-sitosterol) as sold by the company Kaukas. In the case of the use of a mixture of phytosterols, mention may be made, for example, of the product referred to as "Primal" (mainly comprising β-sitosterol, α-sitosterol and campesterol) as sold by the company Kaukas.

The term "ethoxylated derivatives" of phytosterols is understood, according to the invention, to refer to phytosterols, in particular those mentioned above, in which the hydroxyl group has been replaced with a group of formula

H(OCH₂CH₂)ₙO- (I)

in which n represents an integer between 1 and 100 and preferably between 5 and 50. Mention may be made, for example, of the products referred to as "E5", "E10", "E15", "E25" and "E50" sold by the company Kaukas, which are ethoxylated β-sitosterols, the figures 5, 10, 15, 25 and 50 indicating the value of n in the group of formula (I).

Among the pentacyclic triterpenes which can be used in the composition according to the invention, mention may be made in particular of oleananes (with β-amyrine), friedelanes (with friedeline), taraxastanes (with taraxasterol) or ursanes (with α-amyrine).

Among the isoflavones which can be used in the composition according to the invention, mention may be made in particular of daidzein or genistein.

Among the isoflavone glucosides which can be used in the composition according to the invention, mention may be made in particular of daidzin or genistin.

Among the coumestanes which can be used in the composition according to the invention, mention may be made in particular of coumesterol.

Lastly, among the saponosides which can be used in the composition according to the invention, mention may be made in particular of glycyrrhizine from liquorice (Glycyrrhizz glabra), saponosides from marsh pennywort (Centella asiatical, saponosides from mimosa (Mimosa tenuiflora), saponosides from marigold (Calendula officinalis) or saponosides from primula (Primula veris).

According to a particularly preferred embodiment of the invention, the retinoid is retinol and the plant hormone is β-sitosterol or an ethyloxylated derivative thereof.

Preferably, the composition according to the invention has a retinoid concentration of between 0.0001 and 3% by weight and a plant hormone concentration of between 0.0001 and 5% by weight, relative to the total weight of the composition.

The composition according to the invention also comprises at least one cosmetically acceptable excipient, adapted to external topical administration. Excipients adapted to the formulation are known to those skilled in the art and comprise, in particular, thickeners, emulsifiers, preserving agents, dyes, fragrances, etc.

The composition according to the invention can be prepared according to operating conditions which form part of the general knowledge of those skilled in the art.

The composition according to the invention can thus be, for example, in the form of a solution, a gel, a lotion, a cream, an oil-in-water emulsion or a water-in-oil emulsion, or can be combined with any cosmetically acceptable vector, such as liposomes.

Lastly, the composition according to the invention can, needless to say, also contain cosmetic ingredients such as moisturizers, softeners or chemical or organic screening agents.

On account of the activity of the retinoid/plant hormone combination, in particular the combination of retinol with β-sitosterol, on the contents of TIMP inhibitors and of metalloproteinases, the composition according to the invention has an advantageous effect on the main signs of ageing, in terms of efficacy, relative to a composition containing only the retinoid of this combination.

It has thus been observed that the retinoid/plant hormone combination makes it possible at least to potentiate the activity of the retinoid and even, in certain cases, to obtain synergism.

The first effects can be observed after a few weeks of use of the composition and are exerted deep-down. These effects comprise a reduction in the number and/or depth of wrinkles, smoothing of the skin surface and its irregularities, firming of the skin and better moisturization. The composition according to the invention also makes it possible to unify the complexion and/or to prevent or reduce the appearance of age marks, stretch marks and cellulite. The composition according to the invention is particularly suitable for combating wrinkles and the slackening of tissues. It also protects the skin against environmental attack, in particular UV attack, and pollution. It gives a unified appearance and prepares the skin to receive other cosmetic and make-up products.

The composition according to the invention can be used either in the morning and/or the evening on the face or the hands or any other part of the body. On the epidermis of the hands, it will be particularly suitable for reducing hyperpigmentation and for combating the appearance of age marks, as well as for increasing the firmness of the tissues in this region, which have a tendency to become flaccid with age.

The composition according to the invention is particularly suitable for treating the areas around the eyes and the lips, which are very fragile and are highly susceptible to the appearance of wrinkles and slackening. It is particularly well tolerated in this sensitive region, in which, due to the preferably synergistic activity of the retinoid/plant hormone combination, its anti-ageing activity will be exerted after a few weeks of application. This composition makes it possible visibly to reduce the number of wrinkles and bags under the eyes; it firms the particularly sensitive skin around the eyes, the mouth and the neck.

Lastly, the composition according to the invention can also be applied to the exoskeleton, in particular to the hair and the nails. It makes it possible in particular to strengthen the nails by preventing them from becoming brittle, and to improve their appearance in particular by combating the appearance of lines and/or marks.

The present invention also relates to the use of a combination of at least one retinoid, chosen from retinol, esters thereof and retinaldehydes, with at least one plant hormone chosen from the group consisting of phytosterols and ethoxylated derivatives thereof, pentacyclic triterpenes, isoflavones, isoflavone glucosides, coumestanes and saponosides, for the preparation of a composition intended to increase the content of metalloproteinase tissue inhibitor compounds.

In particular, this use is such that the composition prepared is a cosmetic composition or a pharmaceutical composition. When the composition prepared is a pharmaceutical one, it is preferably a dermatological composition.

More particularly, this use is characterized in that the composition prepared is intended to improve the elasticity of the skin and/or is intended for the prevention or treatment of changes in the skin and the exoskeleton associated with ageing.

In particular, the use is characterized in that the retinoid is chosen from retinol and its esters.

Moreover, the use is characterized in that the plant hormone is chosen from phytosterols and ethoxylated derivatives thereof, and is preferably chosen from the group consisting of β-sitosterol, α-sitosterol, γ-sitosterol, β-sitostanol, stigmasterol, stigmastanol, campesterol and campestanol, and ethoxylated derivatives thereof.

Also, as above already disclosed for the composition, the use is characterized in that the retinoid is present at a concentration of between 0.0001 and 3% by weight, and in that the plant hormone is present at a concentration of between 0.0001 and 5% by weight, relative to the total weight of the composition.

Even more particularly, the use is characterized in that the retinoid is retinol and the plant hormone is β-sitosterol or an ethoxylated derivative thereof.

In particular, the use described above makes it possible to prepare a dermo-cosmetic composition intended to improve the viscoelastic properties of the skin and to prevent or reduce the formation of wrinkles.

By "dermo-cosmetic composition", one understand of course a cosmetic composition or a dermatological composition.

Also, the present invention also relates to a method of cosmetic treatment, for increasing the content of metalloproteinase tissue inhibitor compounds, characterized in that a composition, comprising the combination of at least one retinoid with at least one plant hormone, as described above is applied to the skin and/or the exoskeleton.

Lastly, the present invention also relates to a method for the cosmetic treatment of changes associated with ageing of the skin and/or the exoskeleton, characterized in that a composition, comprising the combination of at least one retinoid with at least one plant hormone, as described above is applied to the skin and/or the exoskeleton.

The present invention will now be illustrated with the aid of examples, which should under no circumstances be interpreted as possibly limiting its scope.

### Example 1: Study of the anti-ageing activity of the combination of retinol with β-sitosterol in vitro on human dermal fibroblasts

### 1) Equipment and method

Fibroblasts were isolated from the human dermis from a biopsy from a plastic surgery operation on a 65-year-old patient. They were used at the thirteenth passage and inoculated at about 80,000 cells in 24-well culture plates with 0.5 ml of culture medium supplemented with 10% calf foetal serum and antibiotics.

After 48 hours of inoculation, the cells were treated with the test product at various concentrations of the latter, in a culture medium containing 1% calf foetal serum. Each concentration was tested three times.

After incubation for two days at 37°C (i.e. on "D2" hereinbelow) the number of cells was measured in half of the cultures. The cells of the other half of the cultures were again treated, on D2, with the test product in order to carry out a measurement of the number of cells after incubation for five days at 37°C (i.e. on "D5" hereinbelow).

The measurements of the number of cells, in particular for the cell proliferation study, were carried out with a "Coulter counter" cell counter as sold by the company Coultronics.

The following measurements of the TIMP-1, MMP-1 and MMP-3 were carried out with ELISA kits on the supernatant of the cell culture medium, recovered and stored at -80°C before measuring.

For each series of measurements, the control was untreated cultures or supernatants of untreated cultures.

### 2) Study of the change in TIMP-1

During the *in vivo* ageing, the content of TIMP-1 decreases as already explained hereinabove. The activity of a composition according to the invention on this TIMP-1 content was measured on D2 and D5. The results are given in Table 1 below (the results therein are expressed in nanograms of TIMP-1 per cell).

It can thus be observed that the retinoid/plant hormone combination of a composition according to the invention makes it possible at least to significantly potentiate the activity of the retinoid, if not to provide a synergistic effect, as regards the increase in the content of TIMP-1 inhibitors.

### 3) Study of the change in MMP-1 and MMP-3

During the in *vivo* ageing, the content of MMP-1 (collagenase) and MMP-3 (stromelysines) metalloproteinases increases as already explained hereinabove, which leads to a gradual destruction of the extracellular matrix. The activity of a composition according to the invention on this content of MMP-1 or MMP-3 respectively was measured on D2 and D5. The results are given in Tables 2 and 3 below, respectively (the results are expressed in nanograms of MMP-1 or MMP-3 per cell).

It may thus be observed that the retinoid/plant hormone combination of a composition according to the invention makes it possible to reduce the content of MMP-1 and MMP-3 metalloproteinases. In particular, it should be pointed out that the increase in the content of TIMP-1 inhibitors does not result in any significant increase, by a phenomenon of feedback regulation, of the content of metalloproteinases.

### 4) Study of the cell proliferation

The activity of a composition according to the invention on cell proliferation was measured on D2 and D5. The results (expressed as the number of cells) are given in Table 4 below.

It may thus be observed that the retinoid/plant hormone combination of a composition according to the invention does not result in any significant undesirable decrease in cell renewal.

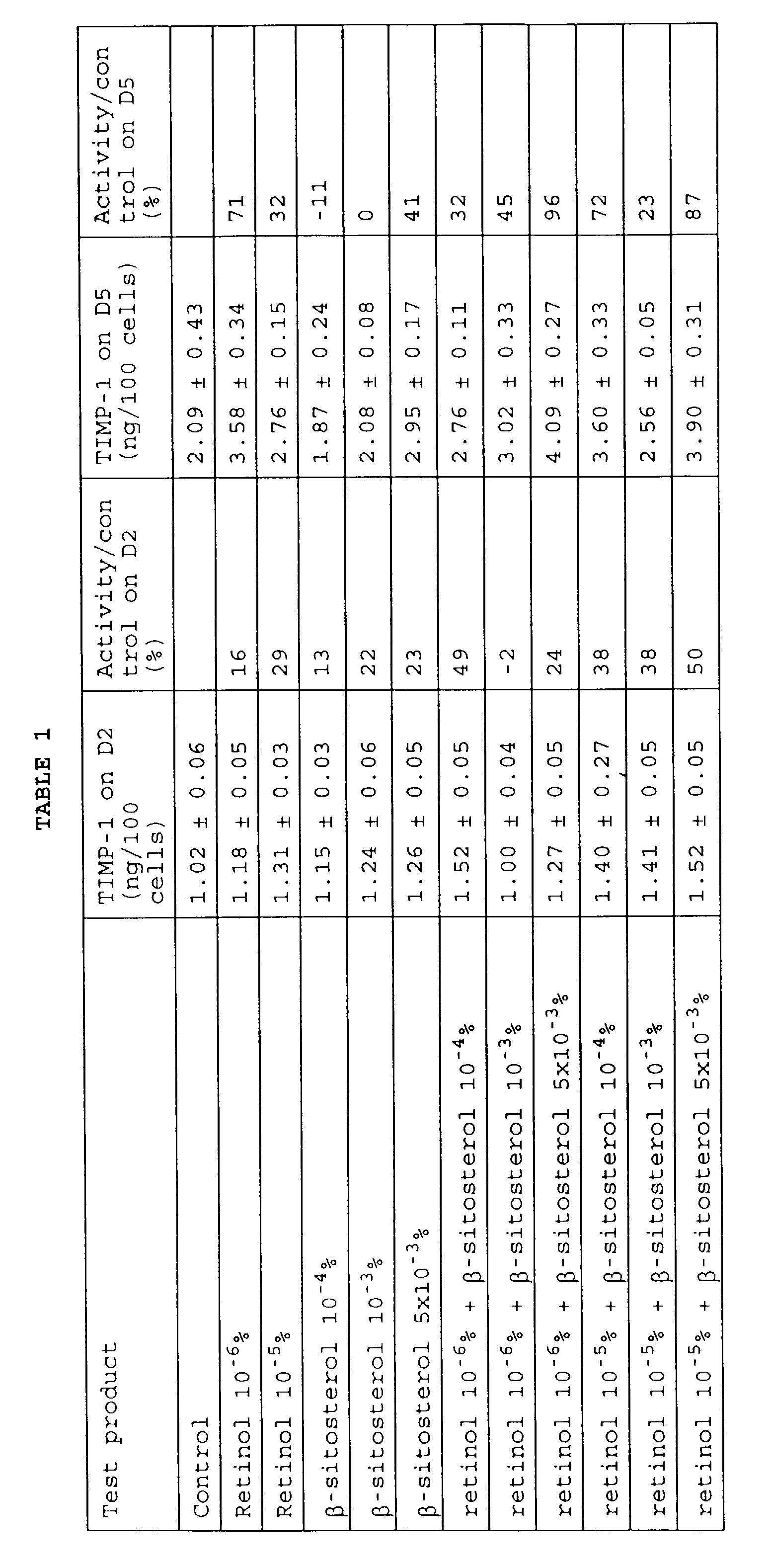

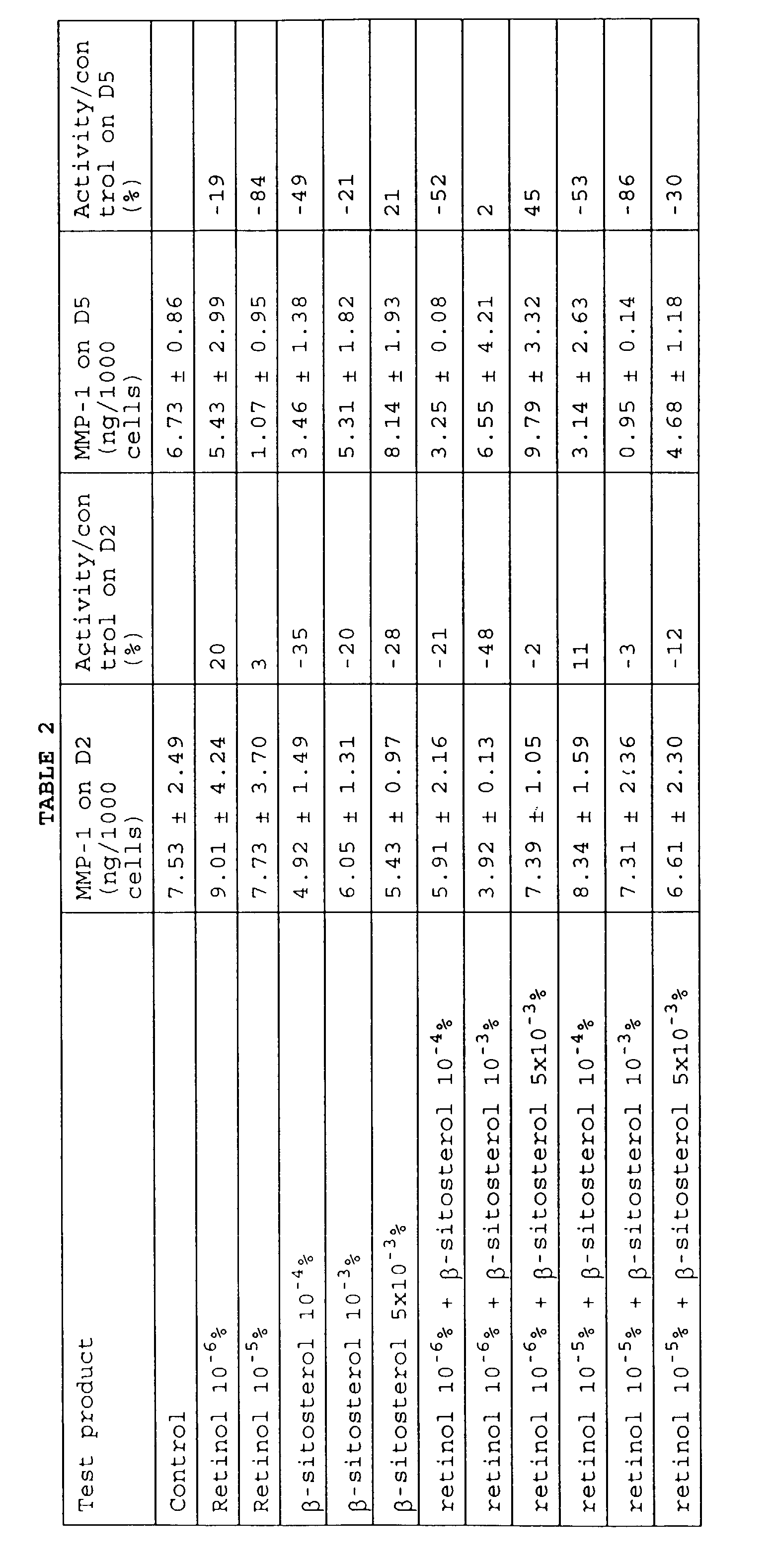

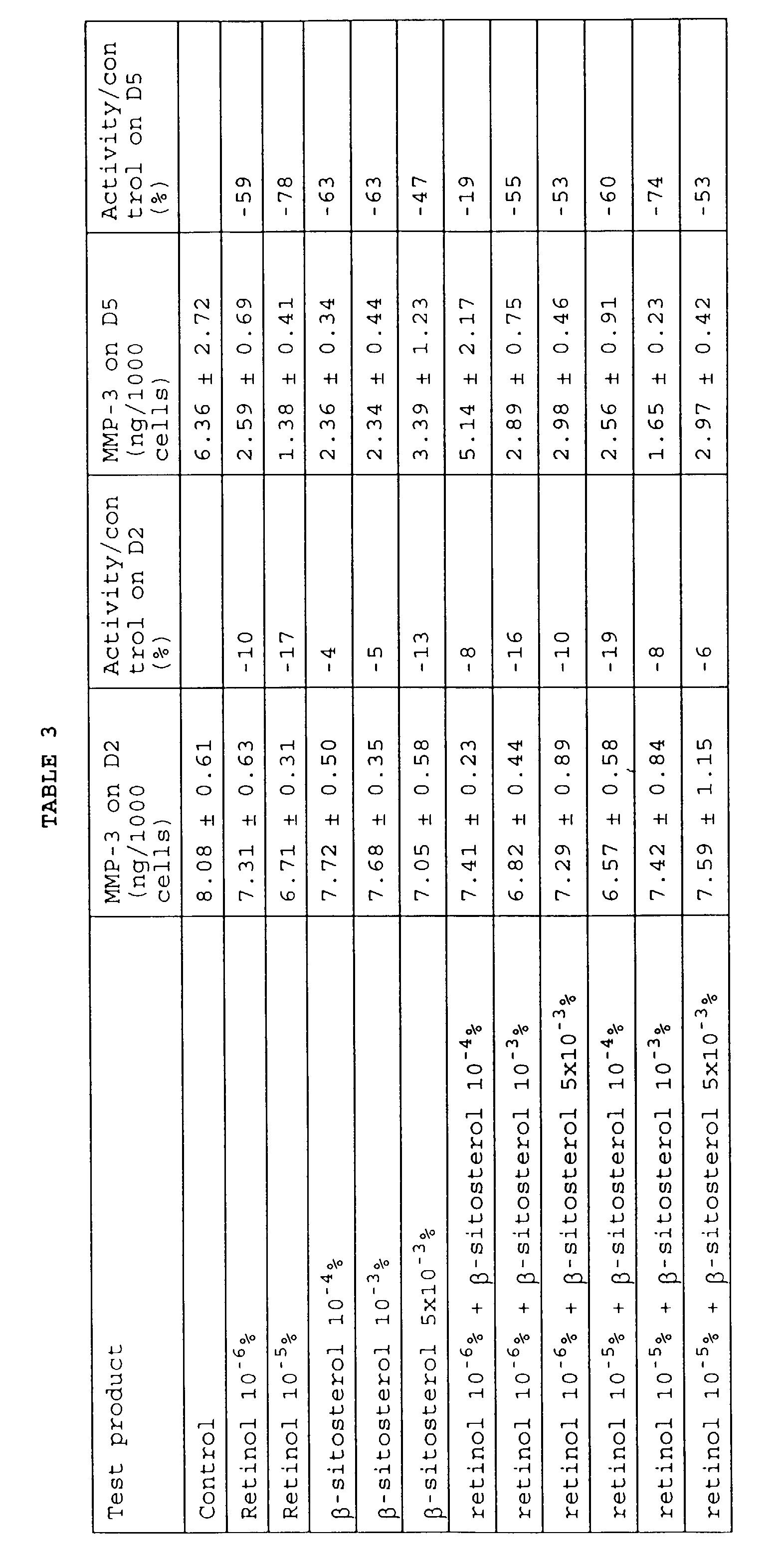

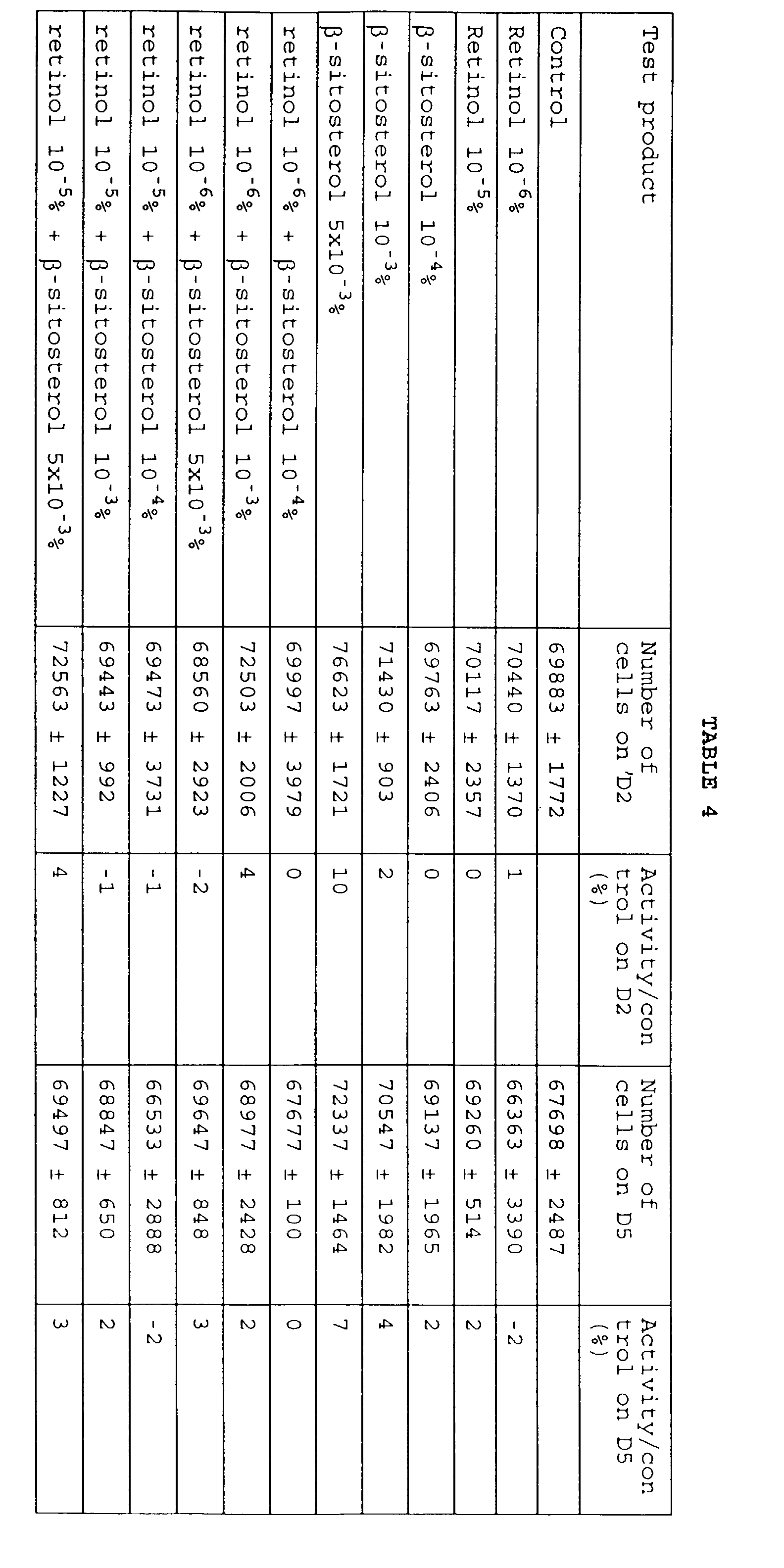

### Example 2: Anti-ageing composition in the form of an oil/water emulsion

| | % |
|---|---|
| - Glycerol | 5.000 |
| - Disodium EDTA | 0.200 |
| - Methyl 4-hydroxybenzoate | 0.200 |
| - propyl 4-hydroxybenzoate | 0.070 |
| - 2-phenoxyethanol | 0.730 |
| - Water | q.s. |
| - Polyacrylamide and C13-14 isoparaffin and laureth-7, such as commercialized under "Sepigel 305" by SEPPIC | 2.000 |
| - Dimethicone | 2.000 |
| - Cetearyl alcohol | 0.500 |
| - Cyclomethicone | 1.500 |
| - Isopropyl palmitate | 2.000 |
| - BHT | 0.100 |
| - Tri(C14-C15)alkyl citrate | 4.000 |
| - 90° alcohol | 6.000 |
| - Hydrogenated polyisobutene | 4.000 |
| - Butylene glycol | 2.000 |
| - Stearic acid | 1.000 |
| - Sodium hydroxide | 0.100 |
| - Retinol | 0.300 |
| - β-sitosterol | 0.500 |
| Total | 100% |

### Example 3: Anti-ageing composition in the form of an oil/water emulsion

| | % |
|---|---|
| - Water | q.s |
| - Carbomer "Carbopol 980" such as | 0.300 |
| commercialized by GOODRICH | |
| - Glycerol | 5.000 |
| - PEG-8 | 2.000 |
| - Disodium EDTA | 0.200 |
| - Sodium hydroxide | 0.135 |
| - Glyceryl stearate/PEG-100 stearate, such as commercialized under "Alarcel 165" by ICI | 5.000 |
| - Cetearyl octanoate/isopropyl myristate, such as commercialized under "Dub liquide" by STEARINERIE DUBOIS | 3.000 |
| - Glyceryl stearate | 3.000 |
| - Stearyl alcohol | 0.500 |
| - Cetostearyl alcohol | 0.500 |
| - Cetyl palmitate | 0.500 |
| - Talc | 0.200 |
| - BHT | 0.100 |
| - Methyl 4-hydroxybenzoate | 0.160 |
| - propyl 4-hydroxybenzoate | 0.056 |
| - 2-phenoxyethanol | 0.584 |
| - C12-15 alkyl benzoate | 4.000 |
| - Dimethicone | 1.000 |
| - Retinol | 0.092 |
| - β-sitosterol | 0.150 |
| | |
| Total | 100% |

### Example 4: Composition according to the invention in the form of a gel

| | % |
|---|---|
| - Glycerol | 5.000 |
| - Disodium EDTA | 0.200 |
| - Methyl 4-hydroxybenzoate | 0.200 |
| - Propyl 4-hydroxybenzoate | 0.070 |
| - 2-phenoxyethanol | 0.730 |
| - Water | q.s. |
| - Polyacrylamide and C13-14 isoparaffin and laureth-7 | 2.000 |
| - Dimethicone | 2.000 |
| - Cetearyl alcohol | 0.500 |
| - Cyclomethicone | 1.500 |
| - Isopropyl palmitate | 2.000 |
| - BHT | 0.100 |
| - Tri(C14-C15)alkyl citrate | 4.000 |
| - 90° alcohol | 6.000 |
| - Hydrogenated polyisobutene | 4.000 |
| - Butylene glycol | 2.000 |
| - Retinol | 0.300 |
| - β-sitosterol | 0.500 |
| Total | 100% |

### Example 5: Composition according to the invention in the form of a lotion (water/oil emulsion)

| | % |
|---|---|
| - Cyclomethicone | 25.000 |
| - Polyglyceryl-2-Sesquiisostearate/Beeswax/ Mineral Oil/Magnesium Stearate/Aluminium Stearate, such as commercialized under "Hostacerin WO" by HOECHST | 12.000 |
| - Phenyl Dimethicone | 6.000 |
| - Dimethicone | 3.000 |
| - Isopropyl Myristate | 3.500 |
| - BHT | 0.050 |
| - Water | q.s.p. |
| - C12-15 Alkyl Benzoate | 3.000 |
| - Methyl 4-hydroxybenzoate | 0.160 |
| - Propyl 4-hydroxybenzoate | 0.056 |
| - 2-phenoxyethanol | 0.584 |
| - β-sitosterol | 0.500 |
| - Disodium EDTA | 0.100 |
| - Retinol | 0.040 |
| | |
| Total | 100% |

## Claims

1. Cosmetic composition, characterized in that it contains a combination of at least one retinoid chosen from retinol, esters thereof and retinaldehydes, with at least one plant hormone chosen from the group consisting of phytosterols and ethoxylated derivatives thereof, pentacyclic triterpenes, isoflavones, isoflavone glucosides, coumestanes and saponosides, and at least one cosmetically acceptable excipient.

2. Cosmetic composition according to claim 1, characterized in that the retinoid is chosen from retinol and its esters.

3. Cosmetic composition according to claim 1 or 2, characterized in that the plant hormone is chosen from phytosterols and ethoxylated derivatives thereof.

4. Cosmetic composition according to any of the preceding claims, characterized in that the plant hormone is chosen from the group consisting of β-sitosterol, α-sitosterol, γ-sitosterol, β-sitostanol, stigmasterol, stigmastanol, campesterol and campestanol, and ethoxylated derivatives thereof.

5. Composition according to any one of the preceding claims, characterized in that the retinoid is retinol and the plant hormone is β-sitosterol or an ethoxylated derivative thereof.

6. Composition according to any one of the preceding claims, characterized in that the retinoid is present at a concentration of between 0.0001 and 3% by weight, and in that the plant hormone is present at a concentration of between 0.0001 and 5% by weight, relative to the total weight of the composition.

7. Use of a combination of at least one retinoid, chosen from retinol, esters thereof and retinaldehydes, with at least one plant hormone chosen from the group consisting of phytosterols and ethoxylated derivatives thereof, pentacyclic triterpenes, isoflavones, isoflavone glucosides, coumestanes and saponosides, for the preparation of a cosmetic composition intended to increase the content of metalloproteinase tissue inhibitor compounds.

8. Use according to Claim 7, characterized in that the cosmetic composition prepared is intended to improve the elasticity of the skin and/or is intended for the prevention or treatment of changes in the skin and the exoskeleton associated with ageing.

9. Use according to claim 7 or 8, characterized in that the retinoid is chosen from retinol and its esters.

10. Use according to any one of claims 7 to 9, characterized in that the plant hormone is chosen from phytosterols and ethoxylated derivatives thereof.

11. Use according to any one of claims 7 to 10, characterized in that the plant hormone is chosen from the group consisting of β-sitosterol, α-sitosterol, γ-sitosterol, β-sitostanol, stigmasterol, stigmastanol, campesterol and campestanol, and ethoxylated derivatives thereof.

12. Use according to any one of Claims 7 to 11, characterized in that the retinoid is retinol and the plant hormone is β-sitosterol or an ethoxylated derivative thereof.

13. Use according to any one of Claims 7 to 12, characterized in that the retinoid is present at a concentration of between 0.0001 and 3% by weight, and in that the plant hormone is present at a concentration of between 0.0001 and 5% by weight, relative to the total weight of the composition.

14. Use according to any one of Claims 7 to 13, for the preparation of a cosmetic composition intended to improve the viscoelastic properties of the skin and to prevent or reduce the formation of wrinkles.

15. Method of cosmetic treatment, for increasing the content of metalloproteinase tissue inhibitor compounds, characterized in that a composition comprising the combination as defined in any one of Claims 7 and 9 to 13 is applied to the skin and/or the exoskeleton.

16. Method for the cosmetic treatment of changes associated with ageing of the skin and/or of the exoskeleton, characterized in that a composition comprising the combination as defined in any one of Claims 7 and 9 to 13 is applied to the skin and/or the exoskeleton.

17. Use of the combination as defined in any one of Claims 7 and 9 to 13, for the preparation of a pharmaceutical composition intended to increase the content of metalloproteinase tissue inhibitor compounds.

18. Use according to Claim 17, characterized in that the pharmaceutical composition is a dermatological composition.

19. Use according to Claim 17 or 18, characterized in that the composition prepared is intended to improve the elasticity of the skin and/or is intended for the prevention or treatment of changes in the skin and the exoskeleton associated with ageing.

20. Use according to any one of Claims 17 to 19, for the preparation of a dermatological composition intended to improve the viscoelastic properties of the skin and to prevent or reduce the formation of wrinkles.
